Europäisches Patentamt

European Patent Office

Office européen des brevets

(19)

(11) Numéro de publication : **0 341 163 B1**

## (12) FASCICULE DE BREVET EUROPEEN

(45) Date de publication du fascicule du brevet :
**03.02.93 Bulletin 93/05**

(51) Int. Cl.$^5$ : **C07C 29/04, C07C 35/08**

(21) Numéro de dépôt : **89420154.0**

(22) Date de dépôt : **27.04.89**

(54) **Procédé de préparation du cyclohexanol.**

(30) Priorité : **02.05.88 FR 8806083**

(43) Date de publication de la demande :
**08.11.89 Bulletin 89/45**

(45) Mention de la délivrance du brevet :
**03.02.93 Bulletin 93/05**

(84) Etats contractants désignés :
**AT BE CH DE ES FR GB GR IT LI LU NL SE**

(56) Documents cités :
**EP-A- 0 010 993**
**EP-A- 0 224 116**
**US-A- 3 644 497**

(73) Titulaire : **RHONE-POULENC CHIMIE**
**25, quai Paul Doumer**
**F-92408 Courbevoie Cédex (FR)**

(72) Inventeur : **Blanchard, Gilbert**
**5, Allée des Acacias Lagny-le-Sec**
**F-60330 Le Plessis Belleville (FR)**
Inventeur : **Costantini, Michel**
**10, rue du Docteur Bonhomme**
**F-69003 Lyon (FR)**
Inventeur : **Lecomte, Jean-Pierre**
**24, rue Boileau**
**F-69006 Lyon (FR)**

(74) Mandataire : **Varnière-Grange, Monique et al**
**RHONE-POULENC CHIMIE Service Brevets**
**Chimie Centre de Recherches des Carrières**
**B.P. 62**
**F-69192 Saint-Fons Cédex (FR)**

## Description

La présente invention a pour objet un procédé de préparation de cyclohexanol potentiellement réalisable par mise en contact de cyclohexène et d'eau en présence d'un acide carboxylique.

Par cyclohexanol potentiellement réalisable on entend un mélange renfermant du cyclohexanol libre et du cyclohexanol combiné sous forme du carboxylate correspondant à l'acide carboxylique présent dans le milieu réactionnel.

Le cyclohexanol est un produit utilisé notamment pour la fabrication du l'acide adipique et les carboxylates de cyclohexyles peuvent être hydrolysés pour former d'une part, une quantité supplémentaire de cyclohexanol et d'autre part, un acide carboxylique qui peut alors être recyclé à la préparation du cyclohexanol potentiellement réalisable.

Diverses méthodes de préparation du cyclohexanol par hydratation du cyclohexène ont été décrites antérieurement qui comprennent la mise en contact du cyclohexène et de l'eau sur divers catalyseurs de nature acide, dont un inventaire est dressé dans le préambule de la demande de brevet EP-A 022 4116.

La demande européenne précitée préconise plus particulièrement de conduire la réaction d'hydratation en présence d'un phénol. Toutefois le développement à l'échelle industrielle des méthodes préconisées antérieurement, dont l'intérêt de principe n'est pas contesté, est largement compromis par les faibles rendements observés en produit recherché.

La demande japonaise publiée sous le n° JA 86/249949 décrit notamment la préparation d'esters de cyclohexyle par réaction du cyclohexène et d'un acide carboxylique en présence d'une zéolithe présentant un rapport des sites acides de surface aux sites acides totaux supérieur à 0.07.

Là encore, si le rendement en ester recherché par rapport au cyclohexène est appréciable, l'intérêt d'une telle technique est limité par le fait que l'on n'obtient pas de cyclohexanol libre, par cette réaction.

Il était donc nécessaire de proposer un procédé de préparation du cyclohexanol potentiellement réalisable qui permette notamment d'obvier au manque d'efficacité des procédés antérieurs et offre la possibilité d'obtenir une proportion notable de cyclohexanol libre dans le mélange réactionnel.

La présente invention a donc pour objet un procédé de préparation de cyclohexanol potentiellement réalisable par réaction du cyclohexène et de l'eau, en présence d'une zéolithe H-ZSM-5 comme catalyseur, caractérisé en ce que la réaction est également conduite en présence d'un acide carboxylique liquide dans les conditions de la réaction, choisi parmi :
- les acides monocarboxyliques aliphatiques saturés,
- leurs homologues comportant un ou plusieurs atomes d'halogène,
- les acides dicarboxyliques aliphatiques saturés,
- leurs homologues comportant un ou plusieurs atomes d'halogène,
- et l'acide benzoïque.

La présente invention a également pour objet un procédé de préparation du cyclohexanol caractérisé en ce qu'il comprend la succession d'étapes suivantes :
a) la mise en réaction du cyclohexène et de l'eau, en présence d'une zéolithe H-ZSM-5 comme catalyseur et d'un acide carboxylique liquide dans les conditions de la réaction pour former un mélange renfermant du cyclohexanol et le carboxylate de cyclohexyle dont le groupe carboxylate correspond à l'acide carboxylique engagé à la réaction,
b) la récupération dudit mélange par tout moyen approprié,
c) l'addition d'eau au mélange, suivi de
d) la distillation du mélange ainsi formé pour récupérer le cyclohexanol.

Il est essentiel, dans le cadre du présent procédé de conduire la réaction en présence d'un acide carboxylique liquide dans les conditions de la réaction.

De préférence, l'acide carboxylique est choisi parmi les acides des classes précitées qui sont liquides à une température inférieure à 120°C.

Parmi les acides carboxyliques convenant à la mise en oeuvre du présent procédé on peut citer : l'acide formique, l'acide acétique, l'acide trifluoroacétique, l'acide monochloroacétique, l'acide butyrique, l'acide isobutyrique, l'acide succinique, l'acide glutarique, l'acide dodécanedioïque et l'acide benzoïque.

Les acides formique, acétique et trifluoracétique conviennent plus particulièrement.

L'usage de l'acide acétique, qui est un produit largemement disponible dans le commerce, est plus particulièrement recommandé.

La quantité précise d'acide carboxylique à mettre en oeuvre peut varier dans de larges limites. De bons résultats sont obtenus lorsque cette quantité est telle que le rapport molaire du cyclohexène à l'acide carboxylique est supérieur ou égal à 0,1, aucun effet positif n'étant constaté lorsque le dit rapport est supérieur à 5. Pour une bonne mise en oeuvre du procédé selon l'invention le rapport sera compris de préférence entre 0,2

EP 0 341 163 B1

et 2.

L'eau peut être présente dans le milieu réactionnel en quantité variable, la quantité précise dépendant pour une large mesure des autres conditions de la réaction. Il a toutefois été constaté que pour une quantité donnée de cyclohexène et d'acide carboxylique, il est avantageux d'utiliser une quantité d'eau telle que le rapport molaire du cyclohexène à l'ensemble (eau + acide carboxylique) soit inférieur à 60 % et, de préférence, supérieur à 1 %. Pour une bonne mise en oeuvre du présent procédé, ledit rapport molaire sera compris entre 2 et 10 %.

La réaction est conduite en présence d'une zéolithe H-ZSM-5 comme catalyseur.

Les zéolithes de série ZSM sont des alumino-silicates cristallins développés et commercialisés par la société Mobil Oil Co qui présentent un rapport silice : alumine de 20 et davantage.

Le type H-ZSM-5 correspond à la forme acide d'une série de produits de structure zéolithique connue.

La température de réaction peut varier dans de larges limites ; toutefois en dessous de 60°C la réaction se déroule lentement, cet inconvénient pouvant être compensé par le recours à une quantité plus importante de catalyseur et au-dessus de 180°C, la sélectivité en produit recherché est abaissée.

Pour une bonne mise en oeuvre du procédé selon l'invention, la température sera comprise entre 90 et 120°C.

La réaction est généralement conduite par contact d'une phase liquide avec le catalyseur solide. Elle peut être conduite à pression atmosphérique ou sous une pression suffisante pour maintenir les réactifs en phase liquide.

En fin de réaction on peut récupérer le mélange de produits (cyclohexanol libre et ester de cyclohexyle correspondant à l'acide carboxylique engagé) et le séparer en ses divers constituants par tout moyen approprié ou bien, ce qui constitue également un objet de la présente invention, récupérer ledit mélange par tout moyen approprié puis après addition d'eau, récupérer du cyclohexanol libre par distillation.

Les exemples ci-après illustrent l'invention, les conventions suivantes y sont utilisées :

ACCH désigne l'acétate de cyclohexyle

C6-OL désigne le cyclohexanol


EXEMPLES :

Exemples 1 à 4 ; essai témoin (a) :

Dans un réacteur en verre de 20 cm3, on charge 0,75 g (8,85 mmol) de cyclohexène, 0,5 g de zéolithe H-ZSM-5 et 5,1 g d'eau et/ou d'acide acétique, le rapport molaire eau/acide étant indiqué dans le tableau (I) ci-après. Après chauffage à 120°C (sauf mention contraire) pendant 4 heures, le mélange est refroidi et on dose par chromatographie le cyclohexanol et l'acétate de cyclohexyle formés.

Les rendements (RR) en sont exprimés par rapport au cyclohexène chargé. Les conditions particulières ainsi que les résultats obtenus figurent dans le tableau (I) ci-après :

3

EP 0 341 163 B1

## TABLEAU I

| Réf | Eau/Acide | RR(%) en C6-OL réalisable | RR(ACCH) % | RR(C6-OL) % |
|-----|-----------|---------------------------|------------|-------------|
| 1 | 0,37 | 77,5 | 65,0 | 12,5 |
| 2 | 2,22 | 71,0 | 33,0 | 38,0 |
| 3 | 5,0 | 63,0 | 23,0 | 40,0 |
| 4 | 13,3 | 38,5 | 13,5 | 25,0 |
| a | * | 15,0 | 0 | 15,0 |

* essai réalisé dans l'eau seule.

EXEMPLES 5 à 7 :

Dans l'appareillage et selon le mode opératoire décrits ci-avant on réalise une série d'essais sur une charge renfermant :
- 9,6 mmol de cyclohexène
- 0,5 g de zéolithe, H-ZSM-5
- 111 mmol d'eau
- 50,7 mmol d'acide acétique
soit un rapport molaire eau/acide de l'ordre de 2,19 et un rapport molaire cyclohexène/(eau + acide) de l'ordre de 5,9 %.

Les conditions particulières ainsi que les résultats obtenus figurent dans le tableau (II) ci-après :

4

TABLEAU II

| Réf | T °C | durée (h) | RR (%) EN C6-OL réalisable | RR(C6-OL) (%) |
|-----|------|-----------|----------------------------|---------------|
| 5 | 90 | 24 | 85,7 | 43,1 |
| 6 | 120 | 4 | 74,2 | 38,1 |
| 7 | 150 | 3 | 51,3 | 26,6 |

EXEMPLES 8 à 12 :

Dans l'appareillage et selon le mode opératoire décrits ci-avant on réalise une série d'essais sur une charge renfermant :
- 9,5 mmol de cyclohexène
- 0,5 g de zéolithe H-ZSM-5
- 111 mmol d'eau
- 51 mmol d'un acide carboxylique dont la nature précise est indiquée dans le tableau (III), ci-après :
soit un rapport molaire eau/acide de 2,18.

Les conditions particulières ainsi que les résultats obtenus en 4 heures de réaction à 120°C sont indiquée dans le tableau (III) ci-après :

5

EP 0 341 163 B1

## TABLEAU III

| Réf | Nature de l'acide | RR (%) en C6-OL réalisable | RR(C6-OL) (%) |
|-----|-------------------|---------------------------|---------------|
| 8 | acétique | 74,2 | 38,1 |
| 9 | trifluoroacétique | 96,5 | 20,3 |
| 10 | formique | 81,7 | 22,0 |
| 11 | valérique | 59,3 | 36,4 |
| 12 | glutarique | 59,2 | 26,0 |

EXEMPLE 13 :

Dans l'appareillage et selon le mode opératoire décrits ci-avant on réalise un essai sur une charge renfermant :
- 18,9 mmol de cyclohexène
- 1 g de zéolithe H-ZSM-5
- 11,1 mmol d'eau
- 22,4 mmol d'acide acétique
(rapport molaire eau/acide = 0,5
rapport molaire cyclohexène/(eau + acide) = 56,3 %).
Les résultats obtenus après 4 h de réaction à 120°C sont les suivants :
RR en C6-OL réalisablel = 47,5 %
RR (C6-OL) = 23,3 %

## Revendications

1. Procédé de préparation de cyclohexanol potentiellement réalisable par réaction du cyclohexène et de l'eau, en présence d'une zéolithe H-ZSM-5 comme catalyseur, caractérisé en ce que la réaction est également conduite en présence d'un acide carboxylique liquide dans les conditions de la réaction choisi dans le groupe constitué par :
   - les acides monocarboxyliques aliphatiques saturés,
   - leurs homologues comportant un ou plusieurs atomes d'halogène,
   - les acides dicarboxyliques aliphatiques saturés,
   - leurs homologues comportant un ou plusieurs atomes d'halogène,
   - et l'acide benzoïque.

6

**2.** Procédé de préparation du cyclohexanol selon la revendication 1, caractérisé en ce qu'il comprend la succession d'étapes suivantes :

a) la mise en réaction du cyclohexène et de l'eau en présence d'une zéolithe H-ZSM-5 comme catalyseur et d'un acide carboxylique liquide dans les conditions de la réaction pour former un mélange renfermant du cyclohexanol et le carboxylate de cyclohexyle dont le groupe carboxylate correspond à l'acide carboxylique engagé à la réaction,

b) la récupération dudit mélange par tout moyen approprié,

c) l'addition d'eau au mélange, suivie de,

d) la distillation du mélange ainsi formé pour récupérer le cyclohexanol.

**3.** Procédé selon la revendication 1 caractérisé en ce que l'acide carboxylique est liquide à une température inférieure ou égale à 120°C.

**4.** Procédé selon l'une quelconque des revendications précédentes caractérisé en ce que l'acide carboxylique est choisi dans le groupe constitué par l'acide formique, l'acide acétique et l'acide trifluoroacétique.

**5.** Procédé selon l'une quelconque des revendications précédentes caractérisé en ce que l'acide carboxylique est l'acide acétique.

**6.** Procédé selon l'une quelconque des revendications précédentes caractérisé en ce que l'acide carboxylique est engagé en quantité telle que le rapport molaire du cyclohexène à l'acide carboxylique est compris entre 0,1 et 5 et, de préférence entre 0,2 et 2.

**7.** Procédé selon l'une quelconque des revendications précédentes caractérisé en ce que le cyclohexène est engagé en quantité telle que son rapport molaire à l'ensemble (eau + acide carboxylique) est inférieur à 60 % et, de préférence, supérieur à 1 %.

**8.** Procédé selon la revendication 8, caractérisé en ce que ledit rapport est compris entre 2 et 10 %.

**9.** Procédé selon l'une quelconque des revendications précédentes caractérisé en ce que la température de réaction est comprise entre 60 et 180°C et, de préférence entre 90 et 120°C.

**Patentansprüche**

**1.** Verfahren zur Herstellung von Cyclohexanol, welches potentiell durchführbar ist durch Umsetzung von Cyclohexen und Wasser in Gegenwart eines Zeolithen H-ZSM-5 als Katalysator, dadurch gekennzeichnet, daß die Umsetzung zugleich in Gegenwart einer unter den Reaktionsbedingungen flüssigen Carbonsäure durchgeführt wird, ausgewählt unter:

den gesättigten, aliphatischen Monocarbonsäuren,

deren ein oder mehrere Halogenatome enthaltenden Homologen,

den gesättigten, aliphatischen Dicarbonsäuren,

deren ein oder mehrere Halogenatome enthaltenden Homologen

und der Benzoesäure.

**2.** Verfahren zur Herstellung von Cyclohexanol gemäß Anspruch 1, dadurch gekennzeichnet, daß es die Abfolge der folgenden Stufen umfaßt:

(a) die Umsetzung von Cyclohexen und Wasser in Gegenwart eines Zeolithen H-ZSM-5 als Katalysator und einer unter den Reaktionsbedingungen flüssigen Carbonsäure zur Bildung eines Gemisches, welches Cyclohexanol und Cyclohexylcarboxylat, dessen Carboxylatgruppe der bei der Reaktion eingesetzten Carbonsäure entspricht, enthält,

(b) die Gewinnung des Gemisches durch jedes geeignete Mittel,

(c) die Zugabe von Wasser zu dem Gemisch und anschließend

(d) die Destillation des so gebildeten Gemisches zur Gewinnung des Cyclohexanols.

**3.** Verfahren gemäß Anspruch 1, dadurch gekennzeichnet, daß die Carbonsäure bei einer Temperatur unterhalb oder gleich 120°C flüssig ist.

**4.** Verfahren gemäß einem der vorhergehenden Ansprüche, dadurch gekennzeichnet, daß die Carbonsäure

7

unter Ameisensäure, Essigsäure und Trifluoressigsäure ausgewählt wird.

**5.** Verfahren gemäß einem der vorhergehenden Ansprüche, dadurch gekennzeichnet, daß die Carbonsäure Essigsäure ist.

**6.** Verfahren gemäß einem der vorhergehenden Ansprüche, dadurch gekennzeichnet, daß die Carbonsäure in einer derartigen Menge eingesetzt wird, daß das Molverhältnis Cyclohexen zur Carbonsäure zwischen 0,1 und 5 und vorzugsweise zwischen 0,2 und 2 liegt.

**7.** Verfahren gemäß einem der vorhergehenden Ansprüche, dadurch gekennzeichnet, daß das Cyclohexen in derartiger Menge eingesetzt wird, daß sein Molverhältnis zur Gesamtheit (Wasser + Carbonsäure) geringer als 60% und vorzugsweise höher als 1% ist.

**8.** Verfahren gemäß Anspruch 7, dadurch gekennzeichnet, daß das Verhältnis zwischen 2 und 10 % beträgt.

**9.** Verfahren gemäß einem der vorhergehenden Ansprüche, dadurch gekennzeichnet, daß die Reaktionstemperatur zwischen 60 und 180°C und vorzugsweise zwischen 90 und 120°C liegt.

## Claims

**1.** Process for the preparation of cyclohexanol potentially being capable of being implemented by reaction of cyclohexene and water in the presence of a zeolite H-ZSM-5 as catalyst, characterised in that the reaction is also conducted in the presence of a carboxylic acid which is liquid under the reaction conditions and chosen from the group consisting of:
- saturated aliphatic monocarboxylic acids,
- their homologues containing one or more halogen atoms,
- saturated aliphatic dicarboxylic acids,
- their homologues containing one or more halogen atoms,
- and benzoic acid.

**2.** Process for the preparation of cyclohexanol according to Claim 1, characterised in that it comprises the succession of following stages:
a) reacting cyclohexene and water in the presence of a zeolite H-ZSM-5 as catalyst and of a carboxylic acid which is liquid under the reaction conditions to form a mixture containing cyclohexanol and the cyclohexylcarboxylate in which the carboxylate group corresponds to the carboxylic acid engaged in the reaction,
b) recovery of the said mixture by any appropriate means,
c) addition of water to the mixture, followed by,
d) distillation of the mixture thus formed to recover the cyclohexanol.

**3.** Process according to Claim 1, characterised in that the carboxylic acid is liquid at a temperature lower than or equal to 120°C.

**4.** Process according to any one of the preceding claims, characterised in that the carboxylic acid is chosen from the group consisting of formic acid, acetic acid and trifluoroacetic acid.

**5.** Process according to any one of the preceding claims, characterised in that the carboxylic acid is acetic acid.

**6.** Process according to any one of the preceding claims, characterised in that the carboxylic acid is engaged in such quantity that the molar ratio of cyclohexene to the carboxylic acid is between 0.1 and 5 and preferably between 0.2 and 2.

**7.** Process according to any one of the preceding claims, characterised in that cyclohexene is engaged in such quantity that its molar ratio to the combination (water + carboxylic acid) is lower than 60 % and preferably higher than 1 %.

**8.** Process according to Claim 7, characterised in that the said ratio is between 2 and 10 %.

9. Process according to any one of the preceding claims, characterised in that the reaction temperature is between 60 and 180°C and preferably between 90 and 120°C.